# EUROPEAN PATENT APPLICATION

(11) **EP 4 101 295 A1**
(43) Date of publication of application: **14.12.2022**
(21) Application number: 22177948.1
(22) Date of filing: 08.06.2022
(51) Int. Cl.: A01K 67/033

(54) **BREEDING SYSTEM OF INSECT LARVAE**

(30) Priority: 10.06.2021 IT 202100015251
(71) Applicant: BugsLife S.r.l., 06031 Bevagna Perugia (IT)
(72) Inventor: The designation of the inventor has not yet been filed
(74) Representative: Caforio, Giuseppe

(57) **Abstract**

The present invention discloses an apparatus and a related method for operating said apparatus aimed to the breeding and / or fattening of insect larvae, in particular soldier fly larvae, for the production of raw materials intended for the feed of farm animals, pets, or humans. Said apparatus is of particular advantage for production on an industrial scale, based on an automated set of operations, with a high breeding density, capable of handling large quantities of raw materials, nevertheless at a low cost of design, implementation and management.

## Description

### Field of application of the invention

Equipment for the breeding of insect larvae on an industrial scale. The breeding of insect larvae can be pursued for multiple purposes, i.g. production of feed materials for farm animals, pets, or humans. Alternatively, insect larvae can be reared as a means for reducing the mass and the volume of organic waste.

### State of the art

It is known that insect larvae can be bred and used as animal feed. There are several approaches for the breeding of insect larvae, which differ in the applied strategy for confining the reared specimens in specific spaces.

For example, many species of insect larvae can be raised in large containers such as deep bins or large shallow boxes.

In particular, soldier fly larvae feed on semi-liquid substrates, hence the need for a container or box. These boxes are generally arranged on a large flat surface, however in order to optimize the stocking density, they can be arranged vertically, that is, by stacking one box on top of the other, in a "column" configuration.

To arrange the boxes in such a "column" configuration (and subsequently to "disassemble" the "column" returning to the single boxes) there are two ways: either human work or the use of robotic automation.

Human work is time-consuming and poses work hazards, such as handling heavy loads.

Robotic automation systems are currently used, for example solutions that make use of sliding rollers and mechanical handling arms in the three spatial dimensions. Such solutions have the drawbacks of requiring expensive design, high costs of operation and maintenance; their implementation is therefore limited only to particularly large breeding facilities.

The main purpose of the present invention is to provide a semi-automated technical solution for the breeding of insect larvae (for example of a soldier fly) that is simple to design, manufacture, operate and maintain.

### Summary of the invention

This and other purposes are achieved according to the technical solution diclosed here and claimed in the section of the claims.

Further advantageous features of the present invention are described here and set forth in the dependent claims.

The present invention is based on the idea that the boxes are stacked vertically to form a "Column of boxes" behaving as a single block, which is moved by a mechanical system (called "Tilting device") which rotates said "Column of boxes" in two dimensions instead of moving in three dimensions (as a mechanical arm would do).

In robotics, beyond a certain threshold of execution speed, the costs for the design and implementation of the technologies increase exponentially. The combination of the "Column of boxes " concept with two-dimensional handling, addresses this problem.

In fact, by moving the boxes in groups, for example in groups of 16 boxes, the handling system can work at low speeds (for example at 1/16 compared to that of a corresponding handling system for single boxes) and be equally competitive.

This allows to reach the working threshold for a good mechanical handling system, without the need to develop high performance (and expensive) solutions.

Said "Column of boxes" is essentially an indissoluble piece formed by the mechanical assembly of a plurality of single cassettes. It could also be a single piece, for example made of cast plastic. In the favourite embodiment, the individual boxes are made of steel, welded together. Alternatively, they could be made of hard plastic or any other material sufficiently strong not to be damaged in mechanical handling. In this regard, steel offers the advantage of high tensile strength and yield stress values, so that it is possible to create columns of boxes starting from thin, light, economical sheets.

The handling system of the "Columns of boxes" involves the use of a mechanical device called "Tilting device", which grips the "Columns of boxes", lifts them, moves them horizontally and rotates them overturning them, in order to empty them of their contents , that is, insect larvae. Said "Tilting device" is basically a mechanical actuator for vertical and horizontal movements and rotation on a vertical plane.

A further advantageous feature of the proposed solution is the sanitization of the boxes. Indeed, the "Tilting device", having the ability to overturn the "Columns of box" downwards, provides the possibility of washing the boxes with liquids when they are overturned; this step is carried out in a dedicated place, called sanitization station.

Once both tasks (emptying and sanitization) have been completed, the "Tilting device" rotates the "Column of boxes" again, repositioning it where it was loaded, thus completing the work cycle.

### List of figures

The main features of the apparatus for the breeding and / or fattening of insect larvae as from this invention are shown in the following figures, provided as an example but not as a limitation, with reference to the attached drawings in which:
Fig. 1, overall view of the apparatus for the breeding and / or fattening insect larvae
Fig. 2, favorite embodiment of the "Tilting device"
Fig. 3, favorite embodiments of the "rigid groups of containment units"
Fig.4, favorite embodiment of the mechanical inserts for gripping said "rigid groups of containment units"
Fig. 5, sanitization station of the "rigid groups of containment units"
Fig. 6, favorite sequence of movements of the "Tilting device"
   (a) 1st Movement: the "Tilting device" lifts a "rigid group of containment units"
   (b) 2nd Movement: the "Tilting device" moves along the track towards the hopper of the emptying station
   (c) 3rd Movement: the "Tilting device" rotates the "rigid group of containment units", overturning the contents inside the hopper of the emptying station
   (d) 4th Movement: the "Tilting device" moves along the track bringing the "rigid group of containment units", just emptied, into the sanitization station
   (e) 5th Movement: the "Tilting device" moves along the track returning to the loading-unloading platform
   (f) 6th Movement: the "Tilting device" rotates the "rigid group of containment units", bringing it to the initial position
   (g) 7th Movement: the "Tilting device" lowers the "rigid group of containment units", placing it in the initial position

### Detailed description of the invention

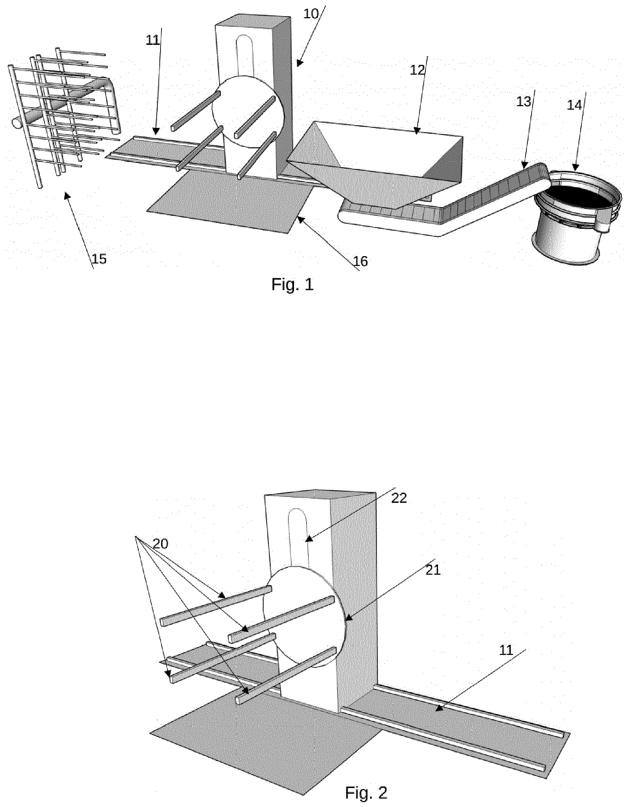

The structure of the apparatus for the breeding and / or fattening insect larvae as from the present invention is schematically shown in Fig. 1, in which the main blocks of the apparatus are shown: a mobile mechanical device (10), called "Tilting device", a track (11) along which the "Tilting device" (10) moves, a hopper-vibrating system (12, 13), a tank for collecting the final farming product (14), a sanitization station (15) for the breeding boxes (30) of the larvae, a loading / unloading platform (16) of said breeding boxes (30).

Said "Tilting device" (10) and said track (11) together create a platform for handling (10, 11) the breeding boxes (30). Said hopper-vibrating system (12, 13) is an emptying station (12, 13) of the breeding boxes (30) for the recovery and separation of larvae and soil.

Fig. 2 shows the favorite embodiment of the "Tilting device" (10), in which the following features can be distinguished: forks (20) for handling the breeding boxes (30), anchored on a mechanical support (21) for synchronous movement of said forks ( 20). A movement actuator in the vertical direction provides for the sliding of said mechanical support (21) along the vertical guides (22) for raising / lowering the breeding boxes (30). An horizontal movement actuator provides for the sliding of said "Tilting device" (10) along said track (11). A rotation actuator with rotation axis perpendicular to the plane defined by horizontal and vertical movements, provides for the overturning of said breeding boxes (30) inside said hopper (12) of said emptying station (12, 13).

Said breeding boxes (30) are organized in "rigid groups of containment units", which can be made as mechanical assemblies of single boxes rigidly fixed to each other to form an indissoluble group, or as monolithic blocks organized in a plurality of compartments.

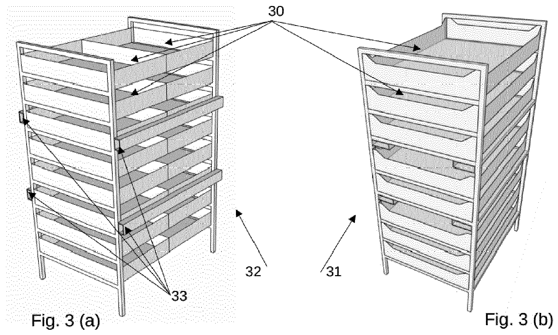

Fig. 3 shows a "column" embodiment (31) of said "rigid groups of containment units" in which said boxes, or compartments (30), have vertical development. A construction option is the "flat" one in which said boxes, or compartments (30), have a horizontal development. The favorite embodiment is the "mixed" one (32) in which said boxes, or compartments (30), have both vertical and horizontal development. Said "rigid groups of containment units" (31, 32) can be made entirely of metal, completely of plastic, or a combination of various materials such as metal for the supporting structural elements, and plastic for the containers. Said "rigid groups of containment units" (31, 32), in order to be handled, are equipped with mechanical inserts (33). The favorite embodiment of said mechanical inserts is that of hollow bars (33) for a safe and stable mechanical attachment of said "rigid groups of containment units" (31, 32) to said "Tilting device" (10).

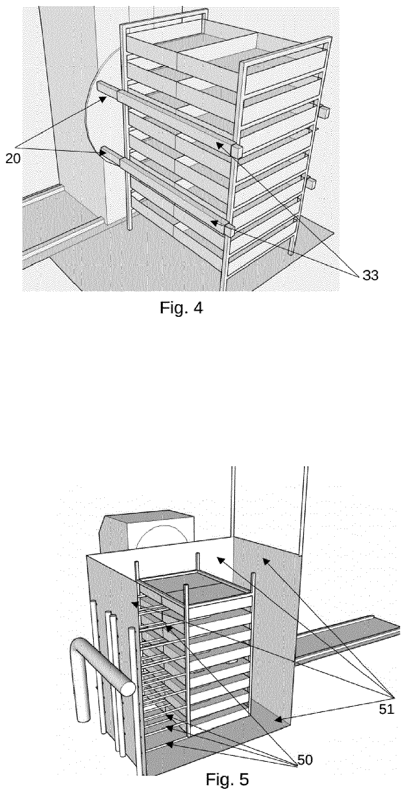

Fig. 4, shows a detail of the coupling between the forks (20) and said hollow bars (33), only two of the four bar-fork couplings are visible in the figure.

Fig. 5 shows an embodiment of said sanitization platform (15). In particular, an ordered system of pipes ending in nozzles (50) is shown which eject liquids under pressure, such as water or water with detergents, also reaching the internal surfaces present between two consecutive rows of "rigid groups of containment units" ( 31, 32). In the favorite embodiment, said ordered system of pipes is enclosed within a containment structure (51), provided with automatic entrance doors, not shown in the figure, in order to prevent splashes of water escaping from the sanitation station (15). Said doors close as soon as a "rigid group of containment units" (31, 32) has got inside the sanitization station (15), then the washing begins. At the end of the water supply, the door open and the "rigid group of containment units" (31, 32) is gripped by the "Tilting device" (10) and taken outside the sanitization platform (15). During cleaning and sanitization, the dirty water can be advantageously recovered on the floor, filtered and reused in order to minimize water consumption.

The breeding and fattening apparatus of insect larvae described here is managed by an operator, who brings each "rigid group of containment units" (31, 32) onto the loading / unloading platform (16), for example by means of a transpallet, and give the start to the automatic emptying and sanitizing sequence. Such sequence is managed by an electronic control unit.

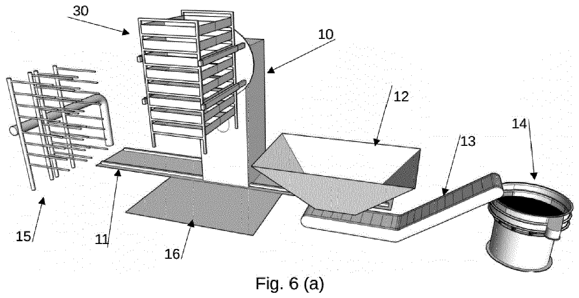

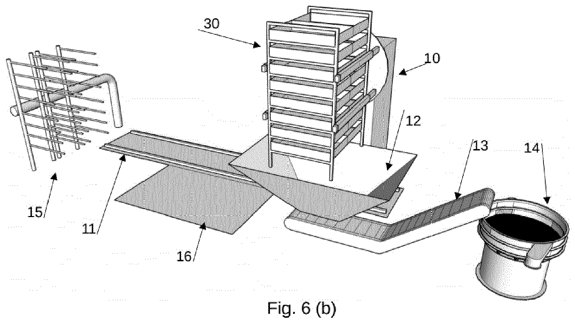

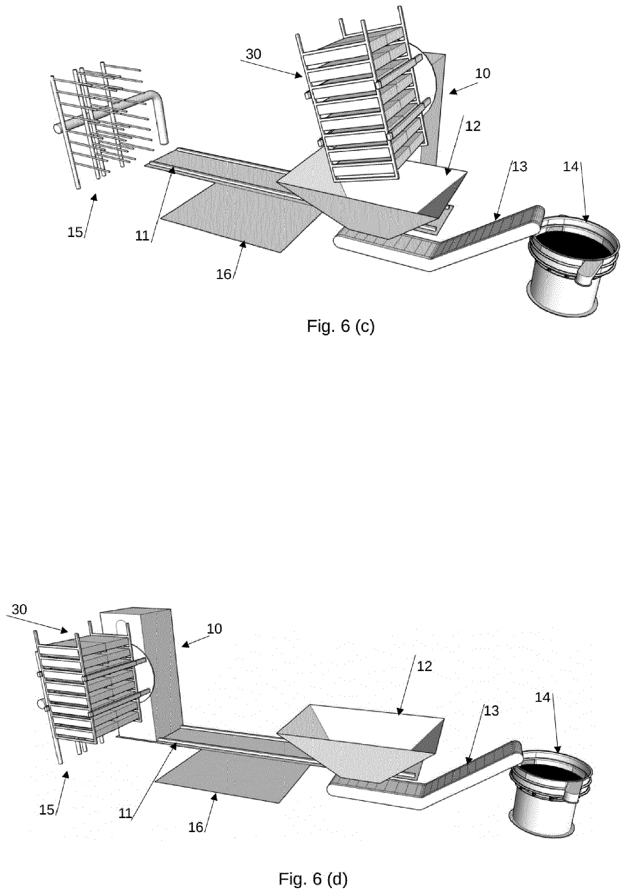

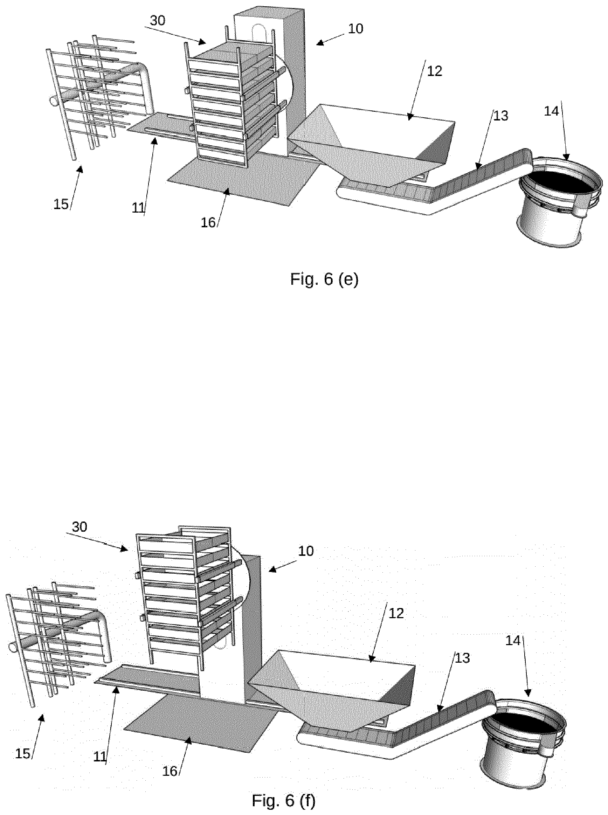

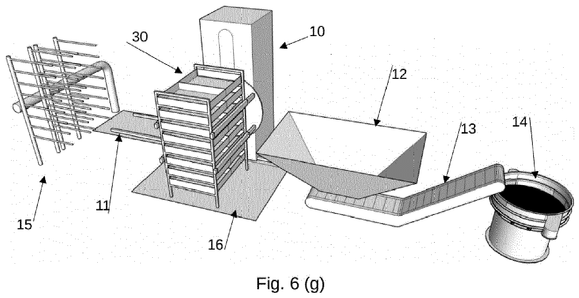

The phases of said emptying and sanitizing sequence are shown in fig. 6 (a), (b), (c), (d), (e), (f), (g). In particular:
a. the "Tilting device" (10) grabs and lifts vertically a "rigid group of containment units" (31, 32)
b. said "Tilting device" (10) travels along the track (11) of the handling platform (10, 11) carrying said "rigid group of containment units" (31, 32) to the hopper (12) of the emptying station (12, 13)
c. said "Tilting device" (10) rotates said "rigid group of containment units" (31, 32) pouring the contents into the hopper (12) of the emptying station (12, 13)
d. said "Tilting device" (10) travels along said track (11) of said moving platform (10, 11), from said hopper (12) to the sanitization station (15), where said "rigid group of containment units" ( 31, 32), now empty, is sanitized. Said "rigid group of containment units" (31, 32) enters upside down so that the washing water does not accumulate inside the boxes, or compartments (30).
e. said "Tilting device" (10) moves said "rigid group of containment units" (31, 32), now cleaned, along said track (11) up to the loading / unloading platform (16), where said "Tilting device" (10 ) implements a rotation movement of said "rigid group of containment units" (31, 32) bringing it back to a non-overturned conformation
f. called "Tilting device" (10) lowers the "rigid group of containment units" (31, 32) vertically, resting it on said loading / unloading platform (16).

## Claims

1. Apparatus for breeding and/or fattening insect larvae comprising a moving system (10, 11) of larvae breeding containers (30), an emptying station (12, 13) equipped with hopper (12) and vibrating station (13) for the recovery and separation of larvae and soil, a sanitization station (15) of the larvae breeding containers (30)
**characterized by the fact that**
- said larvae breeding containers (30) are organized in "rigid groups of containment units" (31, 32), said "rigid groups of containment units" (31, 32) being mechanical assemblies of individual breeding boxes (30) rigidly fixed to each other to form an indissoluble group or monolithic blocks organized in a plurality of compartments (30)
- said "rigid groups of containment units" (31, 32) include inserts (33) for mechanical gripping by a mobile mechanical device
- said moving system (10, 11) comprises a "Tilting device" (10), that is a mobile mechanical device provided with means (20) for the mechanical gripping of said "rigid groups of containment units" (31, 32), able to grab / release, raise / lower, move and overturn said "rigid groups of containment units" (31, 32)
- said moving system (10, 11) further comprises a track (11) for connection between said emptying station (12, 13) and said sanitizing station (15)

2. breeding equipment as from main claim in which said "rigid groups of containment units" (31, 32) are organized in the form of a column (31), in which said boxes or compartments have a vertical development, or are organized in flat form, in which said boxes or compartments have a horizontal development or are organized in a "mixed" form (32) in which said boxes, or compartments (30), have both vertical and horizontal development

3. breeding apparatus as from any of the previous claims, in which said "rigid groups of containment units" (31, 32) can be made entirely of metal, completely of plastic, or a combination of various materials, for example metals for the supporting structural elements, and plastic for the containers

4. breeding apparatus as from any of the previous claims, in which said inserts (33) for mechanical gripping (33) are hollow bars installed on said "rigid groups of containment units" (31, 32), and said mechanical gripping means (20) are forks of the transpallet type installed on the "Tilting device" (10), which can be inserted inside said hollow bars (33) for a safe and stable mechanical gripping

5. breeding equipment as from any of the previous claims, in which said "Tilting device" includes three actuators, for example electric or hydraulic, for the realization of three movements that lie on the same plane:
- a vertical movement for the lifting / lowering of said "rigid groups of containment units" (31, 32)
- a horizontal movement for the displacement of said "rigid groups of containment units" (31, 32) along said track (11) connecting said emptying station (12, 13) and said sanitizing station (15)
- a rotational movement with axis perpendicular to the directions of horizontal movement and vertical movement, for the overturning of said "rigid groups of containment units" (31, 32) within said emptying station (12, 13)

6. breeding apparatus as from any of the previous claims, wherein said sanitizing platform (15) comprises a system of pipes ending in nozzles which eject liquids under pressure, such as water or water with detergents, also reaching the internal surfaces between two consecutive rows of "rigid groups of containment units" (31, 32)

7. breeding apparatus as from any of the previous claims, in which said insect larvae fattened using this apparatus are Soldier Fly larvae

8. Method of mechanical handling of "rigid groups of containment units" (31, 32) as from any of the previous claims
**characterized by the fact that**
it includes the following consecutive phases:
a. the "Tilting device" (10) grabs and lifts vertically a "rigid group of containment units" (31, 32)
b. said "Tilting device" (10) moves along said track (11) of said system system (10, 11) carrying said "rigid group of containment units" (31, 32) to said hopper (12) of said emptying station (12, 13)
c. said "Tilting device" (10) implements a rotation movement of said "rigid group of containment units" (31, 32) pouring the contents into the hopper (12) of the emptying station (12, 13)
d. said "Tilting device" (10) moves along said track (11) of said moving system (10, 11), from said hopper (12) to said sanitization station (15), where said "rigid group of containment units" ( 31, 32), turned upside down and emptied of its content, is sanitized.
e. said "Tilting device" (10) moves said "rigid group of containment units" (31, 32), now cleaned, along said track (11) up to the loading /unloading platform (16), where said "Tilting device" (10) implements a rotation movement of said "rigid group of containment units" (31, 32) bringing it back to a non-overturned conformation
f. called "Tilting device" (10) lowers the "rigid group of containment units" (31, 32) vertically, resting it on said loading /unloading platform (16)
